# EUROPEAN PATENT APPLICATION

(11) **EP 4 184 522 A1**
(43) Date of publication of application: **24.05.2023**
(21) Application number: 21209993.1
(22) Date of filing: 23.11.2021
(51) Int. Cl.: G16H 40/63, G16H 50/20

(54) **A DEVICE AND METHOD FOR PROVIDING CLINICAL INFORMATION OF A SUBJECT**

(71) Applicant: Koninklijke Philips N.V., 5656 AG Eindhoven (NL)
(72) Inventor: BULUT, Murtaza, Eindhoven (NL); BERA, Deep, Eindhoven (NL); MAESSEN, Ralph Theodorus Hubertus, Eindhoven (NL); VAN LIESHOUT, Ron Martinus Laurentius, Eindhoven (NL); SARTOR, Francesco, Eindhoven (NL); VASIST, Santosh Narasimhaswamy, Eindhoven (NL)
(74) Representative: Philips Intellectual Property & Standards

(57) **Abstract**

Provided are concepts for providing clinical information of a subject. In particular, monitor operation setting values are determined for a subject monitoring device, such that relevant information may be provided in an appropriate format. This is achieved by obtaining monitoring data of the subject. Said monitoring data is then used to generate subject monitor parameter values, and is also processed by a condition prediction machine learning model to predict a subject condition. Thus, monitor operation setting values may be determined from the subject monitor parameter values and the predicted subject condition. By determining monitor operation setting values in this way, predictive subject condition information and complex signal-to-signal information may be provided to caregivers in an understandable format.

## Description

### FIELD OF THE INVENTION

The present invention relates to the field of providing clinical information, and particularly to subject monitoring devices for providing said clinical information.

### BACKGROUND OF THE INVENTION

In clinical environments, subject monitors are utilised in order to present vital sign information. Typically, subject monitors are used to display vital signals such as ECG, O₂ saturation, respiration, and blood pressure in real-time, alongside statistical parameters calculated from them, such as mean values, trends, and alarm settings.

The signals and numbers-based view (i.e., signal view) of subject monitors is a highly versatile and capable tool of processing and visualizing real-time changes in individual signals, as well as for calculating and visualizing trends for the past changes in the individual signals. However, it has limited capabilities to display and highlight signal-to-signal interactions both for real-time signals and trend calculations.

Recently, there has been research into using avatars and other infographics (i.e., avatar view) in order to display information related to changes in the real-time signals on subject monitors. Avatar view has been shown to be beneficial for improving the perception of vital signs compared to the conventional monitoring solutions showing signal waveforms and numbers. Moreover, avatar view is linked to reduced workload perception. However, the translation of the interaction and data into an avatar at all times may create a tunnel vision, restricting the caregiver in thinking beyond the data that is given and visualized through the avatar.

Furthermore, the introduction of machine learning based algorithms in healthcare has changed the nature of the information that can be generated. Specifically, vast amounts of personal data has been collected and utilized to build and run machine-learning algorithms using both past and real-time data and signals to evaluate the condition of subjects. This data is typically not visualized by subject monitoring devices, and may not be easily accessible, despite the fact that it may be valuable for patient care. Avatar view may facilitate faster and easier access to such data.

### SUMMARY OF THE INVENTION

The invention is defined by the claims.

According to an aspect of the invention, there is provided a subject monitoring device for providing clinical information of a subject. The device comprises an interface unit configured to obtain monitoring data of the subject from one or more sensors; a data analysis unit configured to generate subject monitor parameter values based on the obtained monitoring data; a first machine learning unit configured to process, with a condition prediction machine learning model, the monitoring data in order to predict a subject condition; and a processing unit configured to determine monitor operation setting values of the subject monitoring device based on the subject monitor parameter values and the predicted subject condition.

Provided by the invention are concepts for the provision of clinical information of a subject. In particular, monitor operation setting values are determined for a subject monitoring device, such that relevant information may be provided in an appropriate format. This is achieved by obtaining monitoring data of the subject. Said monitoring data is then used to generate subject monitor parameter values, and is also processed by a condition prediction machine learning model to predict a subject condition (i.e. a subject's clinical condition). Thus, monitor operation setting values may be determined from the subject monitor parameter values and the predicted subject condition. By determining monitor operation setting values in this way, predictive subject condition information and complex signal-to-signal interaction information may be provided to caregivers in an understandable format, appropriate in the circumstances.

By way of explanation, monitoring data is typically acquired in a medical setting by a sensor arrangement and presented in a real-time signal based view on a subject monitoring device. For example, monitoring data may include a heart rate, a blood pressure, an oxygen saturation, a serum sodium concentration, a respiration rate, etc. Indeed, monitoring data may be any data indicative of a state/condition/health of the subject. As such, typical subject monitoring devices usually present a graph of the recent history of the monitoring data, and a number representing the most recently acquired monitoring data. Embodiments of the present invention determine subject monitor operation settings that may go beyond the typical signal based view in certain circumstances.

Such circumstances are determined by processing the monitoring data to generate subject monitor parameter values (i.e. a current condition of the subject, a trend of a condition of the subject, etc.), while also processing the monitoring data using a machine learning algorithm to generate a prediction of a condition of the subject. In this way, the subject monitor operation settings take into account the current subject condition by analysing the monitoring data, as well as a predicted subject condition that may not be immediately apparent from the monitoring data.

Put another way, a means for controlling monitor operation setting values for a subject monitoring device such that the subject monitor view settings are appropriate for the current condition of the subject, as well as a predicted condition/future condition of the subject may prove very beneficial. In particular, ensuring the monitor operation settings (and therefore the information provided, and the way in which it is presented) are appropriate may save caregiver time, and reduce cognitive load on the caregiver when monitoring the subject. This is achieved by making access, location, visualization, and interpretation of relevant information easier, and thus faster. Indeed, monitor operation settings may also be determined such that key information is less likely to be missed, leading to more positive subject outcomes.

Indeed, it has been realized that processing the monitoring data with a predictive machine learning algorithm may be used to go beyond current information known from the subject monitor parameter values. This may be by producing a prediction of the subject's condition, as well as providing a means to organize, categorize, and visualize all relevant data which may be beyond what is being currently monitored. This may be used to intelligently determine subject monitor operation settings, and thus provide relevant information in an appropriate format. This may improve the speed of access to monitoring information of the subject, while also ensuring that all relevant information is identified and accessible, and presented in a manner that enables faster and more comprehensive analysis and visualization of data.

In some embodiments, the processing unit may further comprise a graphics component configured to generate, based on the determined monitor operation setting values, at least one of an avatar view and a signal view to be presented by the subject monitoring device.

The determined subject monitor settings may be used to generate an avatar view, a signal view, or both. Indeed, the settings may include which of the avatar view, signal view, or a combination to generate. In some cases, the determined monitor operation settings may designate what subject-related information may be presented by the subject monitoring device.

Moreover, it has been demonstrated that one of the signal view and the avatar view can be more advantageous than the other depending on the condition of the subject, and needs of the caregiver. For example, avatar view may prove beneficial when a predicted subject condition cannot be deduced from signals, numbers, or alarms displayed on the subject monitor during a signal view. In this way, situational awareness of the caregiver may be improved. On the other hand, signal view may prove more beneficial when the condition of the subject is unstable, and therefore the caregiver needs to be provided with as much raw data as possible.

In some embodiments, the signal view may include numbers and waveforms representative of the obtained vital sign data, and the avatar view may include at least one of: a graphic representing interactions between different signals of the obtained monitoring data; a graphic representing the predicted subject condition; a graphic representing subject information processed by the condition prediction machine learning model; and a graphic representing a trend of the obtained monitoring data.

Indeed, different forms of signal view and avatar view may also prove advantageous in different circumstances. Such circumstances may be determined by the subject monitor parameter values and the predicted subject condition. In the case that an avatar view is generated, all obtained data used by the machine learning model to predict the subject condition may be used to drive the avatar view.

In some embodiments, the interface may be further configured to obtain subject information, and wherein the first machine learning unit is further configured to process, with the condition prediction machine learning model, the subject information in order to predict the subject condition.

In some embodiments, the subject information may comprise at least one of subject lab data, subject electronic medical records, subject personal health records, subject behavioural data, and subject physiological data.

In this way, a more accurate prediction of a subject condition/state may be obtained, so that information presented (and the way in which the information is presented) to a caregiver is improved. Subject information may provide insights into current and previous conditions and attributes of the subject, and thus may be useful in predicting future conditions and states of the subject. For example, if a subject is over 60 years old, is genetically predisposed to heart problems and has a history of heart conditions, then it may be more likely that the subject would experience another heart-related condition. This information may be difficult to understand, or may not be presented in a signal view, and therefore automatically generating a prediction of a subject condition based on this additional information may be invaluable, as well as the capability to display such information.

In some embodiments, the first machine learning unit may be further configured to process, with the condition prediction machine learning model, the subject monitor parameter values in order to predict the subject condition.

Indeed, the subject monitor parameter values (e.g. the current condition of the subject, and a trend of a subject's condition or vital signs) may provide useful information for predicting a future condition parameter value of the subject. In turn, this may result in more accurate subject condition prediction, and thus more appropriate monitor operation setting values.

In some embodiments, the subject monitor parameter values may include trend data values, and the data analysis unit may be configured to calculate the trend data values based on the monitoring data.

Trend values of the monitoring data may provide important information that is highly relevant to the question of the type and format of the information that would be most appropriate to be provided by the subject monitor display. For example, if monitoring data indicates that a condition of subject has been stable (e.g. no significant variation) for a long period of time, signal view is of little importance. Conversely, avatar view may provide a fast and simple to understand way to present the information when the subject is in a stable state.

Accordingly, by having the subject monitor parameter values include trend data values, more appropriate monitor operation setting values for the context of the subject may be determined.

In some embodiments, the data analysis unit may be configured to: acquire at least one baseline value corresponding to at least part of the monitoring data; and calculate trend data values based on a deviation of the monitoring data from the at least one baseline value over a predetermined length of time.

The at least one baseline value may be a value corresponding to a monitoring data that may be considered typical, or normal for the subject. This may be calculated by the device, be a default value, or be set by a caregiver. For example, if the monitoring data is a heart rate, then a baseline value may be 70 beats per minute. Each baseline value may be dependent on physiology or previous conditions of the subject, or may be a predetermined standard value.

Accordingly, by comparing received monitoring data to the at least one baseline value over a period of time, a deviation/difference between the monitoring data and the at least one baseline value may be determined. This deviation may thus indicate how near or far from the baseline value/normal value that the subject is at, and so may be used to calculate a trend value.

In some embodiments, the subject monitor parameter values may include signal characteristic values, and the data analysis unit may be configured to calculate the signal characteristic values based on the monitoring data.

In some embodiments, the signal characteristic values may comprise at least one of: a maximum value, a minimum value, a variation value or an average value of the monitoring data within a predetermined window of time.

It should be understood that signal characteristic values may be used to summarise the obtained monitoring data, while also being relatively straightforward to generate and provide a fast analysis of the monitoring data. This may prove useful in determining appropriate monitor operation setting values.

In some embodiments, the data analysis unit may be further configured to generate alarm data based on the monitoring data and subject alarm threshold values, and wherein the processing unit is further configured to determine monitor operation setting values based on the alarm data.

For example, it may be beneficial to track the number of times particular alarms are triggered (i.e. when monitoring data exceeds subject alarm threshold values), as well as the deviations from the alarm thresholds. If the number of alarm triggers, or the amount of deviations is greater than defined threshold values, it may be assumed that there are complex signal-to-signal interactions occurring.

The subject alarm threshold values may be calculated from available subject data, or may be set by a caregiver. In any case, the alarm threshold values will preferably be customised to the individual subject, and/or subject condition.

In some embodiments, the monitor operation setting values include at least one of: a user interface mode, a data visualisation mode, a data acquisition mode, a data access mode, a data processing mode, a data storage mode, a software processing requirement mode, and a hardware processing requirement mode.

By way of explanation, the monitor operation setting values are determined based on the subject monitor parameter values and the predicted subject condition. This may mean that a data visualisation/presentation by the monitor is changed, as well as changing data access requirements of the subject monitoring device, such that the subject monitoring device has suitable settings for the circumstances apparent from the subject monitor parameter values and predicted subject condition.

In some embodiments, the data analysis unit may further comprise a second machine learning unit configured to process, with a signal processing machine learning model, the monitoring data in order to generate the subject monitor parameter values.

Indeed, further insights may be generated regarding current subject condition by a machine learning model adapted to process the monitoring data. Indeed, there may be complex interrelations between monitoring data, which may only be easily understood by machine learning. Thus, by generating subject monitor parameter values in this way, monitor operation setting values may be set that provide more relevant information in a more appropriate format.

In some embodiments, the monitoring data may comprise at least one of: signals, numbers, or alarms representative of vital signs of the subject.

Indeed, vital sign data may be any physiological information corresponding to the subject that may provide an indication to the current and/or future condition of the subject. By way of brief example, the vital sign data may include a heart rate, an oxygen saturation, a blood pressure, a respiration rate, etc. However, the skilled person would understand that vital sign data is not restricted to just these.

In some embodiments, the processing unit may be configured to determine the monitor operation setting values responsive to at least one of: the first machine learning unit predicting an updated subject condition; and the obtained monitoring data satisfying a predetermined trigger condition.

Thus, monitor operation setting values may be updated upon an appropriate trigger, ensuring that they remain relevant to the current and/or predicted future condition/state of the subject.

According to another aspect of the invention, there is provided a method for providing clinical information of a subject. The method comprises: obtaining monitoring data of the subject from one or more sensors; generating subject monitor parameter values based on the obtained monitoring data; processing, with a condition prediction machine learning model, the monitoring data in order to predict a subject condition; and determining monitor operation setting values of the subject monitoring device based on the subject monitor parameter values and the predicted subject condition.

According to another aspect of the invention, there is provided a computer program comprising computer program code means adapted, when said computer program is run on a computer, to implement a method for providing clinical information of a subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a typical signal view that may be displayed by a subject monitoring device;
Fig. 2 presents an example of an avatar view that may be displayed by a subject monitoring device;
Fig. 3 shows an illustrative work flow of a method for providing clinical information of a subject according to an exemplary embodiment;
Fig. 4 shows an illustrative work flow of a method for providing clinical information of a subject according to another exemplary embodiment;
Fig. 5 is a simplified block diagram of a subject monitoring device for providing clinical information of a subject according to a further exemplary embodiment; and
Fig. 6 is a flow diagram of a method for providing clinical information of a subject according to another exemplary embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to".

According to proposed concepts, a number of possible solutions may be implemented separately or jointly. That is, although these possible solutions may be described below separately, two or more of these possible solutions may be implemented in one combination or another.

Embodiments of the invention aim to provide concepts for providing clinical information of a subject. In particular, monitor operation setting values are generated for the subject monitoring device, such that relevant information may be provided in an appropriate form. This is achieved by analysing monitoring data of the subject to generate subject monitor parameters, which may be indicative of a current subject condition (i.e. a subject's clinical condition) deducible from a typical signal view of the subject monitoring device. A condition prediction machine learning model is leveraged to predict a (current and/or future) subject condition based on processing the monitoring data. Predicted subject condition and subject monitor parameters may then both be utilised in order to determine monitor operation setting values.

Indeed, embodiments of the proposed invention are concerned with providing the most useful information to caregivers in the corresponding circumstances/scenario. This include leveraging a predicted subject condition, which may not be readily apparent from the raw monitoring data.

Moreover, avatar view as a method of presenting clinical information of a subject has been shown to be beneficial in specific circumstances. Such circumstances may be when complex signal-to-signal interactions must be represented, or when a subject condition is stable. This improves the ease of use of subject monitoring devices for caregivers, reducing cognitive load and the likelihood of misinterpretation. Indeed, avatar view is also useful for representing predicted conditions and their likely origins.

However, avatar view is not desirable in all circumstances, with signal view being very important in certain scenarios. Indeed, in a hypothetical scenario where avatar view is provided as the only source of information for a caregiver, the caregiver may be led to certain conclusions by the way in which the information is presented, therefore limiting their ability to reach certain conclusions. In practice, a caregiver is unlikely to make a conclusion without consulting the signal view. This is because solely relying on avatar view may result in the caregiver missing signal-specific related variations (or noticing such variations late), such as changes in the signal shape characteristics.

Thus, embodiments of the invention may be intended to provide a means by which signal view and/or avatar view may be enabled in appropriate circumstances. Namely, this is achieved by determining monitor view operation values based on a current condition of the subject that is derived directly from monitoring data, and a predicted subject condition based on processing monitoring data (and perhaps other relevant subject data) with a machine learning model.

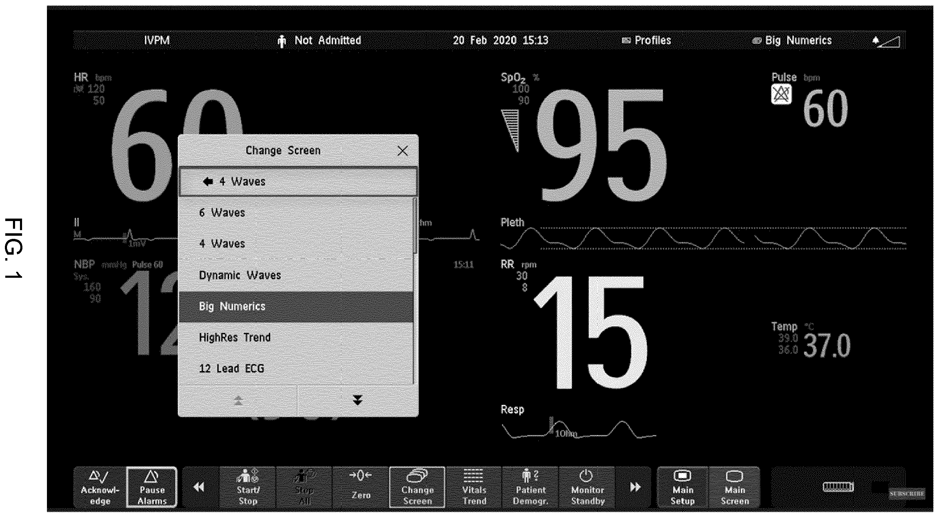

Fig. 1 presents a typical signal view that may be displayed by a subject monitoring device. The signal view includes numbers and signal representative of the current state of the subject.

Overall, the signals and numbers-based view (signal view) of subject monitors is a highly versatile and capable tool of processing and visualizing real-time changes in individual vital sign data signals, as well as for calculating and visualizing trends for the past changes in the individual signals.

However, it is not suitable for displaying information related to predictions of the subjects' future condition. This is because signal-based trend information provides only limited (sensor signal related) information and is only available for individual signals.

Furthermore, the interaction (or inter-relation) between different vital sign data cannot be visualized. Signal view has limited capabilities to display and highlight signal-to-signal interactions both for real-time signals and trend calculations. The complexity of the data (i.e. amount and interactions) and the situation of the subject dictates the signals that the caregiver should focus on. However, due to time constraints, the associated cognitive load, and the tendency to see correlations in data, the caregiver cannot always handle the amount of data provided.

Moreover, the recent increase in availability and application of advanced computational methods such as machine-learning makes it possible to use all types of subject data and signals for the prediction of the subject's future condition. The typical signal view of subject monitoring devices is not capable of displaying all information used for said prediction, such as specific data-processing generated features and signals, as well as signals collected outside the healthcare context, such as user activity data (i.e. step counts, accelerometer signals, sleep data, nutrition data). Additionally, the use of the subject monitor screen during critical situation is aimed for the 'here and now' and thus focused on the signal view. Signal view is not suitable for providing the potential support that predictive elements and/or algorithms can deliver.

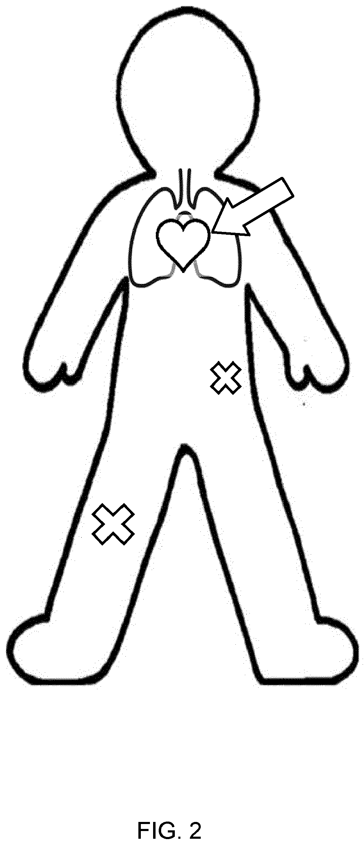

In order to overcome some of the issues that the signal view represents, an avatar view may be provided by the subject monitoring device. An example avatar view is shown in Fig. 2.

The display of an avatar view on the subject monitoring device can be beneficial for improving situational awareness of caregivers, and is suitable for highlighting measured changes in the vital sign data. Avatar-based monitoring (i.e. visual subject technology) improves the perception of vital sign data compared to the conventional monitoring solutions showing signal waveforms and numbers (signal view). Moreover, avatar view is linked to reduced perceived workload by caregivers.

Being able to realize and visualize predictions of a subjects' condition, signal changes, and signal-to-signal interaction is important, as it may improve the speed of access to relevant information, and hence decision-making. Therefore, embodiments of the invention may provide subject monitoring devices that can be adapted for displaying signal information in a different visual manner, and predictive information in a graphical manner (thus going beyond the 'here and now').

Indeed, signal view can be used for displaying predictive information as well. For instance, a stability index can be calculated and displayed. However, displaying a stability index may be difficult to interpret, as it is not clear what it means, which subject data and signals are of most importance, and how it is calculated. Conversely, by utilising an avatar view, some of the limitations linked to displaying a prediction value in signal view may be overcome. For example, the progression of changes in the different signals linked to the calculation of the predicted value can be visualized using the avatar view.

In other words, the predictive information can be also displayed in signal view, by means of a predictive index. Some embodiments of the invention overcome problems associated with this, by displaying the prediction and the subject data influencing the prediction (which can also include data/signals that are not being monitored in real time from the subject) in a graphical manner, by using an avatar view.

Additionally, alarm fatigue from subject monitoring devices is a well-recognized problem. Alarm fatigue results from alarms going off frequently and unnecessarily. Having alarms presented in a variety of different manners (such as using an avatar view and signal view) may be beneficial in reducing the alarm fatigue and the clinical errors associated with it.

However, the translation of the interaction and data into avatar view at all times may create a tunnel vision and may limit the caregiver in thinking beyond the data that is given and visualized through the avatar view. The use of different types of views for the subject monitoring device should be balanced, and the different views should be used in appropriate scenarios, in order to optimize the data uptake by the caregiver and at the same time reduce tunnel-vision. In other words, the monitor operation settings should be altered at the right time to increase the benefit for subject care and to minimize potential clinical error.

By way of brief explanation, clinical errors that may occur during monitoring of a subject include anchoring, availability, framing bias, premature closure, and reliance on authority. Anchoring occurs when initial impressions are relied upon too early in the diagnostic process, which means that clinicians are more like to fail to adjust their initial impressions in light of new information. Availability is when a situation is judged as being more likely or frequent if it easily comes to mind. Framing bias is when a person is swayed by subtleties in how a situation is presented. Premature closure is the inclination to accept a diagnosis before it has been fully verified, essentially believing in a single explanation of a situation without investigating other possibilities. Finally, reliance on authority is when undue reliance is made on authority or technology, Embodiments of the invention aim to minimize these sources of clinical error by adapting the information presented, and the form in which it is presented.

Therefore, embodiments of the invention may automatically and dynamically determine when the provision of an avatar view may be beneficial. The avatar view may then be displayed by the subject monitoring device to visualize real-time signal and trend changes.

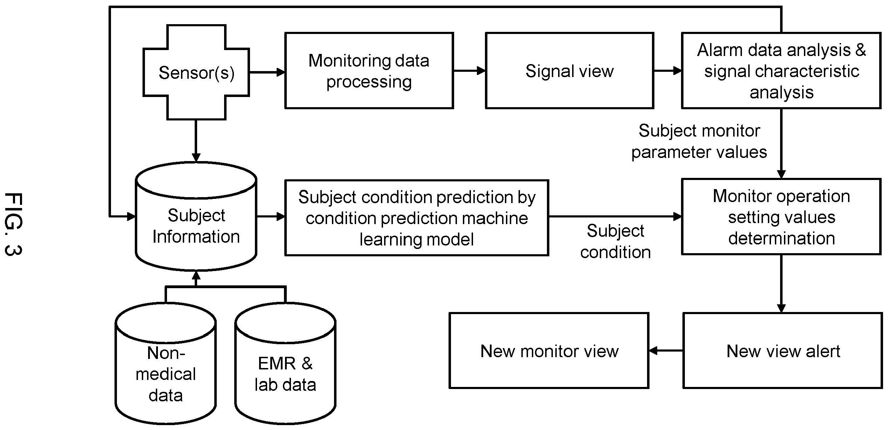

Fig. 3 presents an illustrative work flow of a method for providing clinical information of a subject according to an exemplary embodiment. Overall, the work flow presents the adaptation the subject monitor operation settings to be able to better and more efficiently represent the real-time subject state, as well as to minimize alarm fatigue.

The main elements of the embodiment of the invention presented in Fig. 3 are:
(i) calculating a monitor parameter (subject monitor parameter values) based on trend analysis data, signal data, and alarm data (monitoring data);
(ii) using a condition prediction machine learning model that processes trend analysis data, signal data, and alarm data (and other available subject data) to calculate a prediction parameter (a predicted subject condition);
(iii) using the monitor parameter and the prediction parameter to determine new view settings (monitor operation setting values); and
(iv) generating an avatar view or signal view controlled by both the subject monitor signal analysis and machine learning analysis step, to be presented by the subject monitoring device.

Indeed, further embodiments of the invention provide a functionality of the subject monitoring device to switch between an avatar view and a signal view (i.e. toggle function between avatar and signal views), and a method to analyze long-term and short-term trends of the vital sign data to automatically determine when to switch to avatar or signal view.

More specifically, embodiments of the invention may determine monitor operation setting values that are appropriate in different circumstances (based on monitoring data of the subject). By way of example, a processor may determine updated monitor operation setting values (i.e. for displaying an avatar view) when the subject monitor parameter values and predicted subject condition correspond to the following scenarios:
(a) Missing/Late information case. In particular, when a predicted subject condition cannot be deduced from the signals, numbers, or alarm (monitoring data) currently displayed on the subject monitoring device. That is, when the signal view does not supply enough information to predict the subject condition that may be predicted by machine learning using all subject data. In other words, when the predicted subject condition and subject monitor parameter values indicate that information predicted by the condition prediction machine learning model is not covered by the subject monitor parameter values.

By way of illustrative example, internal bleeding detection is generally based on monitoring heart rate and blood pressure changes. However, these parameters start changing only after 1000 ml of blood is collected, which means that these are late and non-specific indicators. It has been shown that, by using accelerometers to monitor the ballistocardiography (BCG)-like motion of the body, it may be possible to detect internal bleeding earlier, for example when 250 ml of blood has been collected. Now, consider a use case where the machine learning engine will have access to said accelerometer data (which is not currently displayed on the subject monitors). Using this data, it may be possible to predict an internal bleeding event much earlier, before signals such as heart rate and blood pressure start changing.
(b) False information case. When the condition predicted from the signals, numbers, or alarms currently on display on the subject monitor contradicts, or is different to the subject condition predicted by machine learning, using all subject data.
(c) Complex information case. When conditions with complex and varying interactions between vital sign data is predicted by machine learning. In this case, using the avatar view can make multi-signal interactions more understandable. One example of such a case is during a hyperdynamic septic shock event.
(d) Misleading/False signals case. When current monitoring data cannot be trusted. For example, arterial blood pressure may not accurately reflect flow when vascular impedance is abnormal.
(e) Inappropriate/wrong alarm settings, increasing the likelihood of potential false-negative alarms and potential false-positive alarms.
(f) Non-informative alarms. When the alarm provided by signal view is not actionable. It is desirable that the alarms are precise and specific to certain subject conditions in order to reduce alarm fatigue.
(g) Inappropriate or late trend settings. When using the current trend baseline values, the trends will be non-informative or late for the current subject condition.
(h) Missing trend information. Trend indications in signal view may not be shown if, for example, less than 50% of data is available or if the error is larger than, for example, 15% per sample normalized to the used scale.
(i) Significant non-measurement information case. When the most important features predicting the future subject condition are not the vital signals measured by the subject monitor. For example, when a medication, or genetic factors are the main cause, as well as other information that may be present in Electronic Medical Records (EMR) of the subject.

Put a different way, avatar view may be considered superior to signal view during complex signal behaviour and complex signal-to-signal interactions. For example, when there are too many or too fast changes in the vital sign data, or when the interaction between different signals is unclear, and changing monitor view setting values to vary the signal view can have only limited benefit because individual vital sign changes would be either difficult to understand or unreliable. In these instances, a graphical representation that enables observing multi-signal changes simultaneously, and/or at an abstracted level, and/or as their effect analyzed and interpreted by the subject condition predicting machine learning component, may be more informative. In other words, avatar view may enable the visualization of multiple signal changes simultaneously, as well as the output (including the parameters that contribute to the output) of the subject condition predicting machine learning component.

Moreover, avatar view may be considered superior to signal view when the vital sign data is stable. When the vital sign data is stable (i.e., no significant variation from a baseline or range) and within the safe ranges as defined by caregivers, signal view is of little importance. Indeed, in such cases many caregivers do not look at it carefully, or will simply ignore it. However, even in these situations it is important to still observe the subject, and to have a situational awareness, minimizing avoidable clinical errors. To facilitate this, monitor operation setting values may be determined to provide a graphical and somewhat higher-level representation (such as an avatar view). The avatar view may enable the caregiver to be aware of the subject vitals, without spending too much physical and mental effort (which may be the case if they have to look at the signal view).

In some embodiments, the processor may evaluate if any of these conditions apply by the subject monitor parameter values and predicted subject condition. If one or more of the conditions does apply, then a visual (e.g. light, button, color change, etc.) notification indicating the availability of updated monitor operation setting values may be provided. The caregiver can then decide to make use of it. If none of the above conditions apply, then signal view continues and no notification is generated, and no changes to monitor operation setting values are enacted.

Furthermore, if at any point the conditions above become irrelevant or less important (i.e. predictive information becomes less important that real-time information display, such as when the subject's condition becomes very unstable, or deteriorates quickly), then the monitor operation setting values may be automatically switched to a default set of values (i.e. a signal view).

The evaluation of monitor operation setting values may be performed every time the new predictive output (new predicted subject condition), which is significantly different than the previous output, is generated. In other words, the output of a first machine learning unit determines if a new check should be activated or not. Additionally, the checks can be dependent on specific signal, alarm, or trend-based events being triggered.

By way of explanation, the following describes when the above conditions (a)-(i) may be detected by a processor by analysis of the subject monitor parameter values and the predicted subject condition.
(a) When the predicted subject condition indicates a prediction of a significant and important change in the monitoring data, which has not happened yet. This is determined by comparing the changes in the monitoring data associated with the predicted subject condition, with the currently observable vital sign data. For example, using BCG based measurements, internal bleeding may be predicted before heart rate and blood pressure signals start changing abnormally.
(b) In the case that the predicted subject condition and subject monitor parameter values indicate subject conditions that are significantly different, the monitor operation setting values are adapted so that subject condition machine learning output (e.g. subject state prediction, and new alarm suggestions) are visualized.
(c) When the predicted subject condition indicate a prediction of a complex subject condition or when there are many and fast changes in the trend parameters. Complex subject state is a state where vital signs can change in fast and unexpected manner, and when interpretation of changes in one signal depend on changes in other signals. Having a different view (such as an avatar view) can help clinicians in understanding and interpreting the complex variations and interactions in the current signals.
(d) In the case that the predicted subject condition and subject monitor parameter values are different, and the subject monitor parameter values are likely to be erroneous, the monitor operation setting values can be adapted to highlight erroneous signals, and indicate potential source of errors.

The aim is to detect and indicate if some of the real-time monitoring data cannot be trusted. Having early indications of the "bad" monitoring data may help caregivers in considering new measurements. Monitoring data may become erroneous due to misplaced or detached sensors, or due to external factors (such as medication, temperature, user position, interference) influencing signal quality.
(e) The predicted subject condition may represent the alarm settings best fitting to the current subject state/condition. Then using these predicted settings, a number of false-positive or false-negative alarms that can occur in a given time frame can be predicted. The same analyses can be performed for the alarm settings set in the monitor. In case, there is a significant difference between the number of false alarms between two cases, monitor operation setting values can be adapted so that the clinicians are informed of these differences.
(f) The predicted subject condition represents the current subject monitor alarm settings with regard to the subject condition/state the subject condition machine learning algorithm predicted. If it is detected that the alarm is triggered despite a non-significant change in user state, as indicated by the subject monitor parameter values, this may be considered as alarm not being actionable or specific. This information, alongside more actionable alarm suggestions, can be indicated on the monitor.
(g) The predicted subject condition determines the baseline value for the vital signals that are monitored. These baseline values are compared with the trend baseline values provided in the subject monitor parameter values. If there is significant difference, this is indicated in the corresponding view and caregivers may be informed.
(h) If monitoring data is missing, and if trend information from the predicted subject condition is available, then the monitor operation setting values can be updated to present condition prediction machine learning model-based trend information.
(i) The aim is to prevent a tunnel vision, where all the focus is on monitoring data, and other subject information such as medications, genetics, nutrition, motion is not considered. Thus, monitor operation setting values may be determined that alert the caregiver to other causes of the subject condition, which may change their interpretations of the real-time monitoring data.

Moving on, in another embodiment, it is proposed that the subject monitor parameter is a trend parameter. The trend parameter may be discrete and have three values, one representing when the subject is stable, another when there are complex signal to signal interactions, and another when neither condition applies. A trend parameter value may indicate how a condition of the subject has developed in a set time period.

The direction of the trend parameter value may be determined depending on the ascent of the regression line. For example, if ascent of the trend parameter value is greater than 15% then the trend parameter value may be a first value, if greater than 5% then the trend parameter value may be a second value. If less than 5% then the vital sign data may be considered stable.

In an additional embodiment, one can use more detailed analysis of how the monitoring data is varying with respect to the baseline values. In these analyses, the amount (i.e. the difference in values of the baseline set by clinician and the measured value) and frequency (i.e. zero-crossing rate, if baseline is taken as a zero point. In other words, the number of times the baseline is crossed within a given time period) parameters can also be used. If the amount of deviations or the frequency of deviations is greater than set thresholds then complex signal to signal interaction may be assumed.

In yet another embodiment, the alarm information determined from monitoring data can be also used for determination of occurrence of complex signal to signal interaction. Assuming, that the alarm thresholds have been personalized for the current subject, or subject condition, then the number of times particular alarms are triggered may be tracked, as well as the deviations from the alarm thresholds. If the number of alarm triggers, or the number of deviations is greater than defined threshold values, complex signal to signal interactions may be assumed.

Moreover, the subject monitor parameter may include signal characteristics of the monitoring data. This may be used by a processor to calculate indicators of subject condition and stability, according to which monitor operation setting values may be determined.

In particular, the maximum, mean, minimum and variation of monitoring data within a specific time window may be determined. This window can be chosen to be large (i.e. 2 days) or small (i.e. 10 sec). These piecewise approximated values can then be used to compute the number of times the monitored signal has crossed the limit of given thresholds (either set by caregivers for a given subject, or set as default value) to estimate the subject condition (instability, or complex signal behaviour).

By way of example, this may be applied when the monitoring data includes serum sodium concentration values. Hyponatremia is the term for a low sodium concentration in the blood. It is a common electrolyte derangement in the setting of the intensive care unit (ICU). It is generally defined as a sodium concentration of less than 135 mmol/L (135 mEq/L), with severe hyponatremia being below 120 mEq/L. In case of hyponatremia, the subject symptoms can be absent, mild or severe. On the other hand, hypernatremia is the term for a high serum sodium concentration (i.e. >145 mmol/L). Fluctuations in serum sodium concentrations have been independently associated with an increased risk of in-hospital death, even in subjects who remained normonatremic during an ICU stay.

In case of a severe (<120 mmol/L) hyponatremia, life-threatening neurological complications may arise. However, the complication does not only occur at the time of acute fall of plasma sodium levels, but may also arise from overly rapid correction (i.e. the slope or the pattern of the curve is important) of hyponatremia. Additionally, even a mild hyponatremia carries a poor short-term and long-term prognosis across a wide range of conditions. Its multifaceted and intricate physiopathology may seem deterring at a first glance, yet a careful multi-step diagnostic approach may easily unravel the underlying mechanisms and enable physicians to adopt the adequate measures at the subject's bedside. Unless hyponatremia is associated with obvious extracellular fluid volume increase such as in heart failure or cirrhosis, hypertonic saline therapy is the cornerstone of the therapeutic or profound or severely symptomatic hyponatremia.

However, serum sodium concentration values is not the only metric that may be usefully utilised. For example, another signal that may be useful to measure signal characteristics of is oxygen concentration. Indeed, the variability of pulse oximeter plethysmogram (spO2) is useful to assess pulsus paradoxus in subjects with airway obstruction in an ICU.

Accordingly, producing signal characteristics of vital sign data such as serum sodium concentration or oxygen concentration (as a subject monitor parameter value) may prove useful in determining appropriate monitor view setting values.

In embodiments of the invention, the monitor operation setting values may be used by a graphics component to produce a view for display on the subject monitoring device. The view may be generated such that the subject body location is emphasised (using an avatar view) that may be the cause of subject condition changes, such as organ in the subject's body, or position of the subject. Additionally, a transparency view may be generated by the graphics component, where the user could be able to have multiple views (i.e. signal and avatar view) and decide whether to emphasise the avatar view or the signal view more. The user may be enabled to define the transparency. Connected to this embodiment, the avatar view can be made available at all times upon the request of the user, and its emphasis (e.g. size) can change depending on the condition of the subject.

In a more complex embodiment, the graphics component may be configured to generate an avatar movie view, showing the change in the monitoring data, instead of grand averages, and histograms. This may be a beneficial feature, because it may enable quick and efficient visualization of past events and changes in the subject condition. Dynamic changes in the subject conditions may be better observable in the avatar movie view.

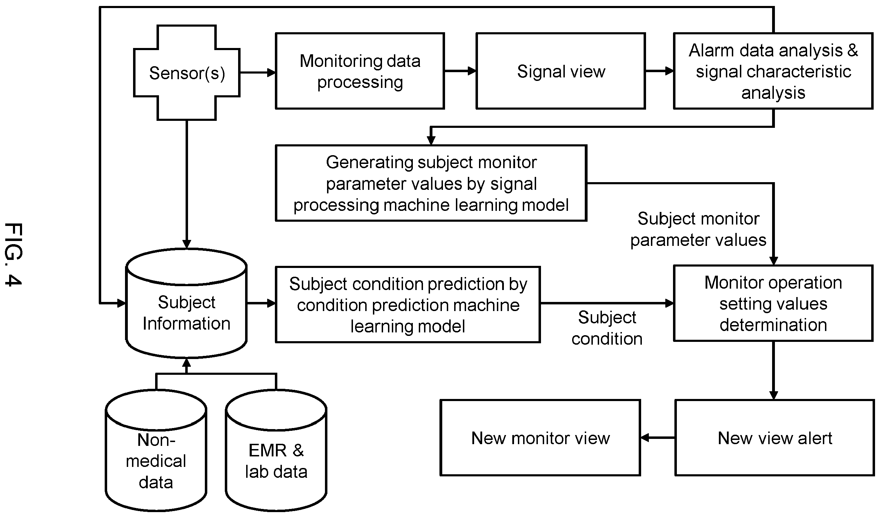

Turning to Fig. 4 illustrative work flow of a method for providing clinical information of a subject according to another exemplary embodiment. This is similar to the workflow shown in Fig. 3, but also includes a machine learning based algorithm integrated in the subject monitors to analyze and interpret the measurement signals/monitoring data.

In the illustrated embodiment, the information generated by the first and second machine learning units (unit 1 is a generic machine learning engine integrated in the subject monitor, and unit 2 is a subject specific machine learning engine) is compared. If the information generated is significantly different, the monitor operation setting values are adapted so that the results of the main unit (unit 2) are shown.

Considering that the main unit (unit 2) would have access to more data, and will be updated more often than unit 1, its results may be more accurate and trustworthy. That is why, in case of significant differences, the monitor operation setting values should be updated to highlight unit 2 results.

It may be argued if unit 2 is more accurate, its output should always be used for adapting the monitor operation setting values. While this may be the case, in practice it may not be always necessary to do so (because the benefit to the subject and caregivers can be limited), and also it can introduce unnecessary cost (e.g. in terms of energy and computational resources) and complexity (in terms of connectivity and data transfer).

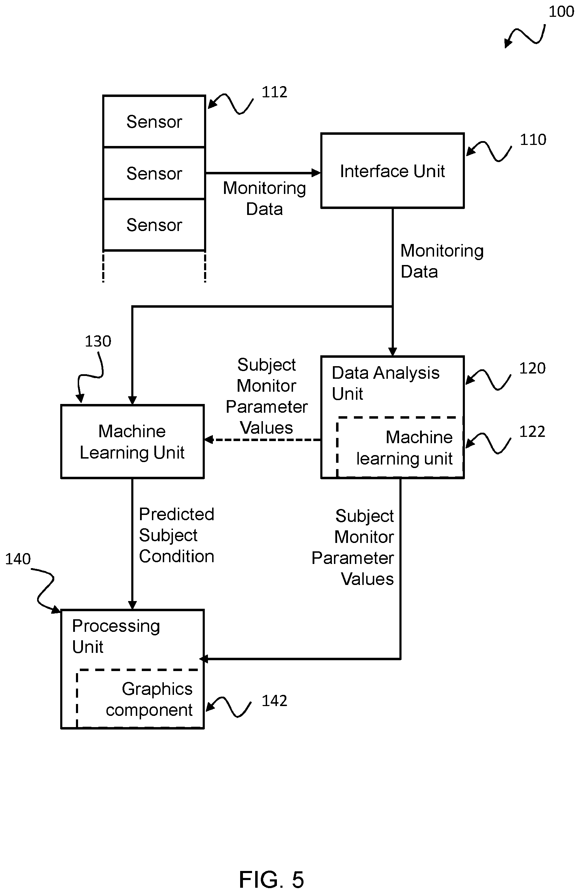

Moving on to Fig. 5, there is presented a simplified block diagram of a subject monitoring device 100 for providing clinical information of a subject. Specifically, the device comprises an interface unit 110, a data analysis unit 120, a first machine learning unit 130, and a processing unit 140. Optionally, the data analysis unit 120 may further comprise a second machine learning unit 122, and the processing unit 140 may further comprise a graphics component 142.

The interface unit 110 is configured to obtain monitoring data of the subject from one or more sensors 112. In other words, the interface unit 110 may be a receiver for signals relating to information about a subject. This may include vital sign data, personal health metrics, ultrasound data, or any other signals that relate to a condition of the subject.

Indeed, the interface unit 110 may be considered as a compiler of information from sensors 112, establishing a common form of the monitoring data for processing by the data analysis unit 120 and the first machine learning unit 130.

The monitoring data may comprise at least one of: signals, numbers, images or alarms representative of vital sign data of the subject. Indeed, in some embodiments, the monitoring data relates to data which is typically received by a subject monitoring device 100 for visualisation on a display. Vital sign data is highly relevant to the condition/state/health of the subject. However, the present invention may also collect additional information relating to the condition/state/health of the subject.

The data analysis unit 120 is configured to generate subject monitor parameter values based on the obtained monitoring data. Indeed, the data analysis unit 120 may receive the monitoring data, and process such data using algorithms known in the art to produce subject monitor parameter values. For example, subject monitor parameter values may encompass values that would typically be presented on a standard subject monitoring device, such as averages, max values, minimum values, variations (i.e standard deviations or variances) and trend values of signals of the monitoring data.

In other words, subject monitor parameter values may be reflective of the condition of the subject that may be directly deducible/derivable from obtained monitoring data.

Taking a specific example, the subject monitor parameter values may include trend data values. Trend data values are a useful tool for determining the current condition of the subject. For example, if a subject's oxygen saturation level is rapidly decreasing, this may indicate that a subject's condition/state/health is deteriorating.

In this case, the data analysis unit 120 may be configured to calculate the trend data values based on the monitoring data. More particularly, the data analysis unit 120 may acquire at least one baseline value corresponding to at least part of the monitoring data. The at least part of the monitoring data may refer to one (or more) signal of the monitoring data, and/or a particular time window of the monitoring data. The baseline value(s) may be set by a caregiver, or be a default value. In some cases, the data analysis unit may calculate the at least one baseline value based on subject information thus providing personalised baseline values. Then, the data analysis unit 120 may calculate trend data values based on a deviation of the monitoring data from the at least one baseline value over a predetermined length of time. In this way, the data analysis unit 1220 may provide a trend value.

In further embodiments, the subject monitor parameter values may include signal characteristic values. Signal characteristic values may provide a simple means to summarise the monitoring data, for less complex subsequent processing. Indeed, signal characteristic values may be directly reflective of an overall condition/state/health of the subject.

In this case, the data analysis unit 120 is configured to calculate the signal characteristic values based on the monitoring data. More particularly, the signal characteristic values may comprise at least one of: a maximum value, a minimum value, a variation value (a variance and/or a standard deviation) or an average value of the monitoring data within a predetermined window of time. However, the skilled person would understand that the signal characteristic values are not restricted to these, and may be any signal characteristic that may provide an indication of a subject's condition.

Furthermore, in a complex embodiment the data analysis unit 120 may further comprise a second machine learning unit 122. The second machine learning unit 122 may be configured to process, with a signal processing machine learning model, the monitoring data in order to generate the subject monitor parameter values.

Indeed, it is increasingly the case that machine learning algorithms are being used to analyse the current condition of the subject. Some subject conditions are not directly deducible/apparent from analysing monitoring data. Machine learning algorithms may be capable of bridging this knowledge gap.

Moreover, this signal processing machine learning algorithm may be general across subjects, and therefore may be trained once across known monitoring data and known subject condition pairs.

Focussing now on the first machine learning unit 130, it is configured to process, with a condition prediction machine learning model, the monitoring data in order to predict a subject condition (i.e. a subject's clinical condition). The condition prediction machine learning model may be trained using known subject condition and known monitoring data pairs. In this way, it may be suitable for predicting the subject condition from monitoring data, drawing conclusions that may be different from subject monitor parameter values directly derivable from monitoring data.

In other words, the condition prediction machine learning model may receive at least the monitoring data as input, and output a subject condition. The output/predicted subject condition may be reflective of a current obscure subject condition, or may indicate a subject condition that is predicted for the future.

However, the condition prediction machine learning model may receive more than just the monitoring data as input, and may also receive other subject information that is relevant to the question of the condition of the subject. Accordingly, the predicted subject condition may take all available data into consideration, providing a more accurate prediction of a subject condition than the subject monitor parameter values.

For instance, the interface 110 may be further configured to obtain subject information. In this case, the first machine learning unit 130 is further configured to process, with the condition prediction machine learning model, the subject information in order to predict the subject condition. Thus, the predicted subject condition may be derived based on inputting monitoring data and subject information into a machine learning model

The subject information may comprise at least one of subject lab data, subject electronic medical records, subject personal health records, subject behavioural data, and subject physiological data. This is data relevant to the question of the predicted condition of the subject. Thus, providing this information to the condition prediction machine learning model may provide a more accurate predicted subject condition. However, the skilled person would understand that data relevant to the predicted condition of the subject is not restricted to the above.

Furthermore, in yet another embodiment, the first machine learning unit 130 may be further configured to process, with the condition prediction machine learning model, the generated subject monitor parameter values in order to predict the subject condition. In this way, the analysed current condition of the subject reflected in the subject monitor parameter values may be taken into account when predicting the subject condition, leading to a more accurately predicted subject condition.

Ultimately, the processing unit 140 is configured to determine monitor operation setting values of the subject monitoring device based on the subject monitor parameter values and the predicted subject condition. As a result, monitor operation setting values are generated that may be used to control the subject monitoring device to present data relevant to the condition of the subject in an appropriate format.

The determined monitor operation setting values may include the view settings of the subject monitoring device, as well as how and what data is being accessed by the subject monitoring device. In other words, as a result, connectivity, data transfer, data access, data acquisition speed, data processing speed, etc. may be set by the monitor operation setting values in order to enable the visualization of relevant data.

Overall, monitor operation settings may consist of settings that control the following parameters of the subject monitoring device: the user interface, the presentation and visualization of data, data access (acquisition, access, processing, and storage), software processing requirements (software version, functionalities, etc.), and hardware processing requirements (memory, hard drive, etc.), as well as permissions, protocols, and guidelines that determine data access and processing.

Thus, the monitor operation settings may need to be adapted so that the information may be presented by the subject monitoring device. In one embodiment, the link between signal/avatar view and the settings mentioned above can be determined based on pre-specified rules and/or real-time data processing needed for enabling and modifying the mentioned views.

Moreover, it may be necessary to display data other than sensor data (i.e. other than monitoring data) because the condition prediction machine learning model may make use of all subject data, and if there would be need to display other subject data, there may need for a connection established to a sources where such data resides. To account for these factors, it may be monitor operation setting values, may include view, connectivity, access, storage, memory, processing settings.

Indeed, the processing unit 140 may further comprise a graphics component 142 configured to generate, based on the determined monitor operation setting values, at least one of an avatar view and a signal view to be presented/enabled by the subject monitoring device. The format in which relevant data is presented is key for positive clinical outcomes. Thus, the generation of a signal view, an avatar view, or a combination of the two is key for presenting the information appropriately.

By way of explanation, the signal view may include numbers and waveforms representative of the obtained monitoring data. In other words, signal view is the typical view shown by standard subject monitoring device. For example, said devices tend to present an average of each signal, perhaps accompanied by a graph and/or histogram of the recent values. This may prove beneficial in certain circumstances, such as during a rapidly deteriorating subject condition, or when an overview of raw data is required.

Avatar view may include at least one of a graphic representing interactions between different signals of the obtained monitoring data, a graphic representing the predicted subject condition, a graphic representing subject information processed by the condition prediction machine learning model, and a graphic representing a trend of the obtained monitoring data. Put another way, avatar view presents an infographic of either a summary of relevant data, a predicted condition of the subject, or any other means to represent complex signal-to-signal interactions. Overall, avatar view may provide a simple means to understand complex data, or data that is otherwise impractical to be presented by signal view.

In particular, avatar view may include any type of data used by the condition prediction machine learning model (monitoring data, subject information, etc.). For example, if lab data used by the condition prediction machine learning model is relevant to the predicted condition of the subject, this may be presented in avatar view.

In other words, avatar view may be driven by the data deduced from the condition prediction machine learning model, which may be used to make the depicted avatar change.

Additionally, the data analysis unit 120 may be further configured to generate alarm data based on the monitoring data and subject alarm threshold values. Alarm values may be when a caregiver would typically be alerted to the change in a condition of the subject due to a change in monitoring data values. In this case, the processing unit 140 may be further configured to determine monitor operation setting values based on the alarm data. For instance, if there have been over a certain number of alarms trigger in a predetermined window, then a change in monitor operation setting values may be beneficial. The subject alarm threshold values may be set for the subject by the data analysis unit automatically, or may be set by a caregiver.

Finally, the processing unit 140 may be configured to determine the monitor operation setting values responsive to at least one of the first machine learning unit 130 predicting an updated subject condition, or the obtained monitoring data satisfying a predetermined trigger condition. In this way, whenever a potential switch in monitor operations setting values may be required to keep the display up-to-date, the processor reassesses the predicted subject condition and subject monitor parameter values.

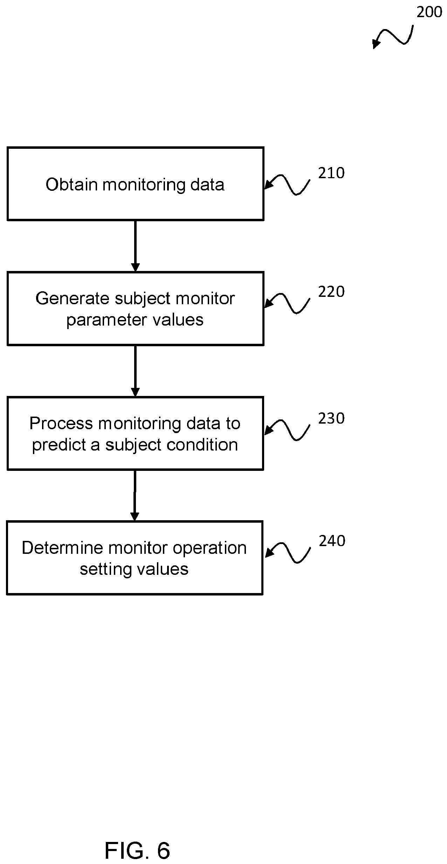

Fig. 6 is a flow diagram of a method 200 for providing clinical information of a subject.

At step 210, monitoring data of the subject is obtained from one or more sensors. This step may be conducted continuously during operation of a subject monitoring device. Indeed, monitoring data may relate to one or more signals relevant to the condition of the subject.

At step 220, subject monitor parameter values are generated based on the obtained monitoring data. Indeed, the subject monitor parameter values may be analogous to the information typically provided by a vital sign monitor. In other words, the subject monitor parameters comprise information relating to the current state of the subject.

Indeed, step 220 may comprise many sub-steps comprising different algorithms performed on the monitoring data, or part of the monitoring data.

At step 230, the monitoring data is processed with a condition prediction machine learning model, in order to predict a subject condition.

Put another way, the monitoring data (and, in some embodiments, other subject-related data) is input to a condition prediction machine learning model. The subject condition machine learning model then predicts the current and/or future state of the subject.

At step 240, monitor operation setting values of the subject monitoring device are determined based on the subject monitor parameter values and the predicted subject condition. Indeed, this essentially compares the current subject condition which is apparent from the monitoring data, and the predicted subject condition. This may give valuable insights into whether there exist complex signal-to-signal interactions, missing data, etc.

The method 200 may comprise a further step (not shown here), where the monitor operation setting values are used to generate a view of the subject monitoring device.

A single processor or other unit may fulfil the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage.

Any reference signs in the claims should not be construed as limiting the scope.

## Claims

1. A subject monitoring device (100) for providing clinical information of a subject, the device comprising:
an interface unit (110) configured to obtain monitoring data of the subject from one or more sensors (112);
a data analysis unit (120) configured to generate subject monitor parameter values based on the obtained monitoring data;
a first machine learning unit (130) configured to process, with a condition prediction machine learning model, the monitoring data in order to predict a subject condition; and
a processing unit (140) configured to determine monitor operation setting values of the subject monitoring device based on the subject monitor parameter values and the predicted subject condition.

2. The device of claim 1, wherein the processing unit (140) further comprises a graphics component (145) configured to generate, based on the determined monitor operation setting values, at least one of an avatar view and a signal view to be presented by the subject monitoring device.

3. The device of claim 2, wherein the signal view includes numbers and waveforms representative of the obtained monitoring data, and wherein avatar view includes at least one of: a graphic representing interactions between different signals of the obtained monitoring data; a graphic representing the predicted subject condition; a graphic representing subject information processed by the condition prediction machine learning model; and a graphic representing a trend of the obtained monitoring data.

4. The device of any of claims 1 to 3, wherein the interface (110) is further configured to obtain subject information, and wherein the first machine learning unit (130) is further configured to process, with the condition prediction machine learning model, the subject information in order to predict the subject condition.

5. The device of claim 4, wherein the subject information comprises at least one of subject lab data, subject electronic medical records, subject personal health records, subject behavioural data, and subject physiological data.

6. The device of any of claims 1 to 5, wherein the first machine learning unit (130) is further configured to process, with the condition prediction machine learning model, the generated subject monitor parameter values in order to predict the subject condition.

7. The device of any claims 1 to 6, wherein the subject monitor parameter values include trend data values, and the data analysis unit (120) is configured to calculate the trend data values based on the monitoring data, and preferably wherein the data analysis unit is configured to:
acquire at least one baseline value corresponding to at least part of the monitoring data; and
calculate trend data values based on a deviation of the monitoring data from the at least one baseline value over a predetermined length of time.

8. The device of any of claims 1 to 7, wherein the subject monitor parameter values include signal characteristic values, and the data analysis unit (120) is configured to calculate the signal characteristic values based on the monitoring data, and preferably wherein the signal characteristic values comprising at least one of: a maximum value, a minimum value, a variation value, or an average value of the monitoring data within a predetermined window of time.

9. The device of any of claims 1 to 8, wherein the data analysis unit (120) is further configured to generate alarm data based on the monitoring data and subject alarm threshold values, and wherein the processing unit (140) is further configured to determine monitor operation setting values based on the alarm data.

10. The device of any of claims 1 to 9, wherein the monitor operation setting values include at least one of: a user interface mode, a data visualisation mode, a data acquisition mode, a data access mode, a data processing mode, a data storage mode, a software processing requirement mode, and a hardware processing requirement mode.

11. The device of any of claims 1 to 10, wherein the data analysis unit (120) further comprises a second machine learning unit (122) configured to process, with a signal processing machine learning model, the monitoring data in order to generate the subject monitor parameter values.

12. The device of any of claims 1 to 11, wherein the monitoring data comprises at least one of: signals, numbers, or alarms representative of vital sign data of the subject.

13. The device of any of claims 1 to 12, wherein the processing unit (140) is configured to determine the monitor operation setting values responsive to at least one of:
the first machine learning unit (130) predicting an updated subject condition; or
the obtained monitoring data satisfying a predetermined trigger condition.

14. A method (200) for providing clinical information of a subject, the method comprising:
obtaining (210) monitoring data of the subject from one or more sensors;
generating (220) subject monitor parameter values based on the obtained monitoring data;
processing (230), with a condition prediction machine learning model, the monitoring data in order to predict a subject condition; and
determining (240) monitor operation setting values of the subject monitoring device based on the subject monitor parameter values and the predicted subject condition.

15. A computer program comprising computer program code means adapted, when said computer program is run on a computer, to implement the method of claim 14.
